# EUROPÄISCHE PATENTSCHRIFT

(11) **EP 2 335 778 B1**
(45) Veröffentlichungstag und Bekanntmachung des Hinweises auf die Patenterteilung: **05.10.2016**
(21) Anmeldenummer: 10194107.8
(22) Anmeldetag: 08.12.2010
(51) Int. Cl.: A61N 5/10

(54) **Strahlentherapievorrichtung**
Radiotherapy device
Dispositif de thérapie par rayonnement

(30) Priorität: 18.12.2009 DE 102009058777
(43) Veröffentlichungstag der Anmeldung: 22.06.2011
(73) Patentinhaber: Carl Zeiss Meditec AG, 07745 Jena (DE)
(72) Erfinder: Kleinwächter, Timo, 72525, Münsingen (DE); Röder, Norman, 73430, Aalen (DE); Fuchs, Holger, 73431, Aalen (DE); Schneider, Frank, Dr., 68159, Mannheim (DE); Wenz, Frederik, Prof. Dr., 69120, Heidelberg (DE)
(74) Vertreter: Müller & Schubert

(56) Entgegenhaltungen:
- EP-A2- 1 033 147
- WO-A2-99/42149
- DE-A1- 3 823 604
- DE-A1-102008 030 590
- US-A- 5 528 652
- US-A- 5 851 171
- US-A- 5 947 891
- US-A- 6 159 139
- US-B1- 6 285 735
- US-B2- 8 303 476

## Beschreibung

Die vorliegende Erfindung betrifft eine Strahlentherapievorrichtung für die Radiotherapie.

Die vorliegende Erfindung liegt insbesondere auf dem Gebiet der Strahlentherapie, insbesondere im Zusammenhang mit der Bestrahlung von Tumoren und dergleichen.

Strahlentherapievorrichtungen bestehen in der Regel aus einer Strahlungsquelleneinrichtung, bei deren Betrieb Strahlung, beispielsweise Röntgenstrahlung, entsteht. Über eine Sondeneinrichtung oder ein Strahlungsquellenelement wird die erzeugte Strahlung an den zu bestrahlenden Ort geführt. Dazu werden in der Regel so genannte Applikatoreinrichtungen eingesetzt.

Eine Applikatoreinrichtung besteht beispielsweise aus einem Applikatorelement, das zur Aufnahme einer Sondeneinrichtung oder eines Strahlungsquellenelements einer Strahlentherapievorrichtung ausgebildet ist. Das bedeutet, dass das Sondenelement oder das Strahlungsquellenelement der Strahltherapievorrichtung in das Applikatorelement eingeführt, beispielsweise eingeschoben, wird. Derartige Applikatorelemente sind im Stand der Technik bereits bekannt.

Beispielsweise ist in der Patentanmeldung DE 10 2008 030 590 A1 der Anmelderin eine Applikatoreinrichtung beschrieben, bei der das Applikatorelement über einen Grundkörper verfügt, der aus einer Reihe verschiedener Bereiche besteht. Ein erster Bereich wird durch den Fußbereich gebildet. Er dient zur Aufnahme mindestens eines Bauelements einer Strahlentherapievorrichtung, beispielsweise wenigstens eines Bauelements einer Sondeneinrichtung oder eines Strahlungsquellenelements, die einen Bestandteil der Strahlentherapievorrichtung darstellt. An den Fußbereich schließt sich ein weitgehend zylindrischer Führungsbereich an, der zur Aufnahme und Führung einer Sondeneinrichtung oder einer Strahlungsquelleneinrichtung dient. Zwischen Fußbereich und Führungsbereich ist ein Übergangsbereich vorgesehen. Schließlich verfügt der Applikator an seinem distalen Ende über einen Kopfbereich, in dem insbesondere die für eine Bestrahlung benötigte Strahlung freigesetzt wird.

Beim Betrieb der Strahlentherapievorrichtung entsteht in der Sondeneinrichtung oder im Strahlungsquellenelement Strahlung, beispielsweise Röntgenstrahlung, die zumindest im Führungsbereich der Applikatoreinrichtung freigesetzt wird. Mit der Applikatoreinrichtung kann erreicht werden, dass Körpergewebe direkt am Ort eines Tumors bestrahlt werden kann.

Die bekannte Applikatoreinrichtung wird dazu eingesetzt, um eine Bestrahlung in eng begrenzten Körperbereichen, insbesondere in Kanälen zu ermöglichen.

In der US 6,285,735 B1 wird eine Einrichtung für die Strahlentherapie beschrieben, die ein Applikatorelement aufweist, welche an einer Strahlungsquelle befestigt wird. Das Applikatorelement weist einen Führungsbereich auf, an dessen Ende sich eine Endkappe befindet. Über die Endkappe wird die Röntgenstrahlung zur Bestrahlung einer Körperoberfläche abgegeben. Das Applikatorelement kann am distalen Ende eine Befestigungseinrichtung aufweisen, über die eine Applikatorelementverlängerung, an welcher die Endkappe angeordnet ist, mit dem Applikatorelement verbunden werden kann.

Zylindrische Applikatoreinrichtungen können aber auch dazu dienen, Körperhöhlen zu bestrahlen. Wenn eine Applikatoreinrichtung beispielsweise zylindrisch ausgebildet ist, kann sie insbesondere dazu dienen, Körperhöhlen zu bestrahlen, die in etwa zylindrisch geformt sind, wie beispielsweise die Vagina oder das Rektum: Dabei wird eine zylindrische Applikatoreinrichtung in die Körperhöhle eingeführt. Dann wiederum wird ein Strahlungsquellenelement in die Applikatoreinrichtung eingeführt und damit der Zielbereich bestrahlt. Oftmals werden mit der Strahlungsquelleneinrichtung auch verschiedene Punkte in der Applikatoreinrichtung angefahren, um damit eine größere Fläche bestrahlen zu können. Das Anfahren von verschiedenen Punkten erfolgt bisher meist mit aufwändigen Mechanismen. Weiterhin besteht die Gefahr, dass die Applikatoreinrichtung verrutschen kann, so dass nicht mehr die gewünschte Region bestrahlt wird.

Der vorliegenden Erfindung liegt deshalb die Aufgabe zugrunde, eine Strahlentherapievorrichtung der eingangs genannten Art derart weiterzubilden, dass ein unerwünschtes Verrutschen der Applikatoreinrichtung, insbesondere während des Bestrahlungsvorgangs, vermieden werden kann.

Diese Aufgabe wird erfindungsgemäß gelöst durch die Strahlentherapievorrichtung mit den Merkmalen gemäß dem unabhängigen Patentanspruch 1. Weitere Merkmale und Details der Erfindung ergeben sich aus den Unteransprüchen, der Beschreibung sowie den Zeichnungen.

Der vorliegenden Erfindung basiert auf dem Grundgedanken, dass die Applikatoreinrichtung nunmehr wenigstens zwei Applikatorelemente aufweist, wobei ein Applikatorelement als äußeres Applikatorelement ausgebildet ist, während wenigstens ein weiteres Applikatorelement als inneres Applikatorelement ausgebildet ist, wobei das innere Applikatorelement in dem äußeren Applikatorelement angeordnet wird und insbesondere während des Bestrahlungsbetriebs in diesem angeordnet ist.

Erfindungsgemäß wird ein Strahlentherapievorrichtung für die Radiotherapie mit Röntgenstrahlung wobei die Strahlentherapievorrichtung zur Bestrahlung der Vagina oder des Rektums eingesetzt wird bereitgestellt, mit einer Strahlungsquelleneinrichtung und mit einer Applikatoreinrichtung zur Aufnahme einer Sondeneinrichtung oder eines Strahlungsquellenelements der Strahlungsquelleneinrichtung, wobei die Applikatoreinrichtung als Vaginalapplikator oder als Rektalapplikator ausgebildet ist, wobei die Applikatoreinrichtung ein erstes Applikatorelement aufweist, welches als Innenapplikatorelement ausgebildet ist, wobei die Sondeneinrichtung oder das Strahlungsquellenelement der Strahlungsquelleneinrichtung im Innenapplikatorelement vorgesehen ist, wobei das Innenapplikatorelement einen Fußbereich, über den das Innenapplikatorelement an der Strahlungsquelleneinrichtung befestigt ist, einen sich an den Fußbereich anschließenden zylindrischen Führungsbereich und eine dem Fußbereich gegenüberliegende Spitze aufweist, wobei die Applikatoreinrichtung ein zweites Applikatorelement aufweist, das in Bezug auf das Innenapplikatorelement als Außenapplikatorelement ausgebildet ist, wobei das Außenapplikatorelement einen Aufnahmeraum zur Aufnahme des Innenapplikatorelements aufweist, wobei das Außenapplikatorelement dazu einen zylindrischen Führungsbereich aufweist und wobei der zylindrische Führungsbereich des Innenapplikatorelements in den zylindrischen Führungsbereich des Außenapplikatorelements eingeführt ist, und wobei das Innenapplikatorelement an seiner Spitze und das Außenapplikatorelement an seiner Spitze geschlossen sind.

Insbesondere ist erfindungsgemäß vorgesehen, dass das Innenapplikatorelement einen Fußbereich aufweist, über den das Innenapplikatorelement an der Strahlungsquelleneinrichtung befestigbar ist. Somit kann das Innenapplikatorelement über seinen Fußbereich an der Strahlungsquelleneinrichtung befestigt und fixiert werden.

Eine derart ausgebildete Strahlentherapievorrichtung ist besonders geeignet, die gestellte Aufgabe zu lösen. Insbesondere kann mit einer solchen Strahlentherapievorrichtung verhindert werden, dass diese während des Bestrahlungsbetriebs verrutschen kann, so dass die eingangs geschilderten Nachteile vermieden werden können.

Die Applikatoreinrichtung besteht zunächst aus wenigstens einem Applikatorelement, welches dazu dient, eine Sondeneinrichtung oder ein Strahlungsquellenelement einer Strahlentherapievorrichtung aufzunehmen. Erfindungsgemäß soll es sich bei diesem Applikatorelement um ein Innenapplikatorelement handeln. Das innere Applikatorelement wird über eine Sondeneinrichtung oder ein Strahlungsquellenelement geschoben.

Zusätzlich ist gemäß der vorliegenden Erfindung nunmehr vorgesehen, dass die Applikatoreinrichtung noch über ein weiteres Applikatorelement verfügt. Hierbei handelt es sich um ein Applikatorelement, das in Bezug auf das Innenapplikatorelement ein Außenapplikatorelement darstellt. Die erfindungsgemäße Applikatoreinrichtung besteht somit aus einem inneren und einem äußeren Applikatorelement.

Das äußere Applikatorelement kann in eine zu bestrahlende Körperhöhle eingebracht und anschließend beispielsweise fixiert werden. Mittel einer Überprüfungsmethode, beispielsweise mittels Ultraschall oder Röntgen, kann dann beispielsweise kontrolliert werden, ob das äußere Applikatorelement an der richtigen Stelle platziert ist. Das innere Applikatorelement, das über die Sondeneinrichtung oder das Strahlungsquellenelement geschoben worden ist, wird in das äußere Applikatorelement eingeführt. Anschließend kann die Strahlendosis appliziert werden.

Zur Aufnahme des inneren Applikatorelements weist das Außenapplikatorelement einen Aufnahmeraum auf, der zur Aufnahme von zumindest einem Bestandteil des Innenapplikatorelements ausgebildet ist. Weiterhin ist das Innenapplikatorelement in einer Weise ausgebildet, dass es zumindest bereichsweise in das Außenapplikatorelement einführbar und im Betrieb der Applikatoreinrichtung eingeführt ist.

Bevorzugt ist dabei, dass das Innenapplikatorelement einen Fußbereich aufweist, über den das Innenapplikatorelement an der Strahlungsquelleneinrichtung befestigbar ist. Dadurch wird ermöglicht, dass die Sondeneinrichtung oder das Strahlungsquellenelement und das Innenapplikatorelement gemeinsam im Außenapplikatorelement bewegt werden können.

Erfindungsgemäß handelt es sich bei der Applikatoreinrichtung um eine Applikatoreinrichtung für die Radiotherapie. Erfindungsgemäß wird die Applikatoreinrichtung im Zusammenhang mit der Bestrahlung der Vagina oder des Rektums eingesetzt, so dass es sich in einem solchen Fall bei der Applikatoreinrichtung um einen Vaginalapplikator oder einen Rektalapplikator handelt.

Grundsätzlich ist die Erfindung nicht auf bestimmte Ausführungsformen für die Applikatorelemente beschränkt. Wichtig ist lediglich, dass der innere Applikator im äußeren Applikator so platziert werden kann, dass die gewünschte Strahlungsdosis zielgerichtet an den Ort der Bestrahlung gebracht werden kann. Beispielsweise kann das Außenapplikatorelement und/oder das Innenapplikatorelement in einer wie einleitend beschriebenen Weise ausgebildet sein. Erfindungsgemäß ist vorgesehen, dass das Innenapplikatorelement und das Außenapplikatorelement jeweils einen zylindrischen Führungsbereich aufweisen und dass der zylindrische Führungsbereich des Innenapplikatorelements in den zylindrischen Führungsbereich des Außenapplikatorelements einführbar ist.

Vorzugsweise kann die Applikatoreinrichtung eine Befestigungseinrichtung und/oder eine - insbesondere lösbare - Fixiereinrichtung aufweisen. Beispielsweise kann vorgesehen sein, dass die Befestigungseinrichtung und/oder die Fixiereinrichtung mit dem Innenapplikatorelement oder dem Außenapplikatorelement verbunden ist/wird. Nachdem das äußere Applikatorelement in die zu bestrahlende Körperhöhle eingeführt worden ist, kann es beispielsweise über die Befestigungseinrichtung am Patienten befestigt und in der gewünschten Position fixiert werden. Beispielsweise kann dies mittels einer geeigneten Riemenkonstruktion realisiert werden, wobei die Erfindung nicht auf das genannte Beispiel beschränkt ist. Vorteilhaft wird zunächst das Außenapplikatorelement in die Körperhöhle eingeführt und am Patienten befestigt und fixiert. Zusätzlich kann die richtige Positionierung noch kontrolliert werden, beispielsweise mittels Ultraschall oder Röntgen. Danach erst wir das Innenapplikatorelement in das Außenapplikatorelement eingebracht.

Vorteilhaft kann das Innenapplikatorelement verschiebbar und/oder drehbar im Außenapplikatorelement ausgebildet oder angeordnet sein. Eine Ausgestaltung, bei der das innere Applikatorelement in dem äußeren Applikatorelement, insbesondere in Längsrichtung, verschoben werden kann und/oder bei der das innere Applikatorelement im äußeren Applikatorelement gedreht werden kann, hat insbesondere den Vorteil, dass auch noch weitere Strahlungsdosen an anderen Stellen appliziert werden können. Das Außenapplikatorelement muss dabei nicht bewegt werden. Es reicht lediglich aus, dass das Innenapplikatorelement, und damit die darin befindliche Strahlensonde oder das Strahlungsquellenelement, mittels der Verschiebung an die gewünschte Stelle gebracht werden können.

Vorteilhaft kann wenigstens ein Abstandhalterelement zur Fixierung des Innenapplikatorelements in einer bestimmten Position vorgesehen sein. Ein solches Abstandhalterelement ist insbesondere dann von Vorteil, wenn das Innenapplikatorelement im Außenapplikatorelement verschiebbar angeordnet beziehungsweise ausgebildet ist. Dabei kann mittels des Abstandhalterelements vorteilhaft die zu verstellende Distanz eingestellt werden. Beispielsweise kann vorgesehen sein, dass wenigstens ein Abstandhalterelementim Aufnahmeraum des Außenapplikatorelements vorgesehen ist. Wenn das Außenapplikatorelement einen Fußbereich und einen Führungsbereich aufweist, kann beispielsweise vorgesehen sein, dass wenigstens ein Abstandhalterelement im Fußbereich und/oder im Führungsbereich vorgesehen ist. Weiterhin kann vorgesehen sein, dass das Abstandhalterelement vor dem Außenapplikatorelement vorgesehen ist, beispielsweise vor dessen Einführungsöffnung. Je nach Ausgestaltung kann vorgesehen sein, dass wenigstens ein Abstandhalterelement vor und/oder in dem Außenapplikatorelement vorgesehen ist.

Die vorliegende Erfindung ist nicht auf bestimmte Ausführungsformen von Abstandhalterelementen beschränkt. Vorzugsweise, jedoch nicht ausschließlich, ist das Abstandhalterelement in einer Weise geformt, dass ein Herausziehen des inneren Applikatorelements unnötig ist. Dadurch wird vermieden, dass das äußere Applikatorelement verrutschen kann. Um dies zu erreichen, kann das Abstandhalterelement beispielsweise als Halbkreis oder als 5/8-Kreis ausgebildet oder ausgeformt sein.

Um verschiedene Strahlungsdosen an unterschiedlichen Stellen applizieren zu können kann vorteilhaft auch vorgesehen sein, dass die Applikatoreinrichtung zwei oder mehr Innenapplikatorelemente mit unterschiedlichen Längen, insbesondere mit unterschiedlichen Längen der zylindrischen Führungsbereiche, aufweist. Insbesondere dann, wenn das äußere Applikatorelement ausreichend fest befestigt ist, so dass ein Verrutschen nicht zu befürchten ist, können solche inneren Applikatorelemente mit verschiedenen Längen zum Einsatz kommen. Eine solche Ausgestaltung hat, im Vergleich zu einer Ausführung mit Abstandhalterelementen, den Vorteil, dass das Innenapplikatorelement und das Außenapplikatorelement derart eng ineinander geführt werden können, dass kein, oder aber zumindest nur ein sehr geringes Luftvolumen zwischen den beiden Applikatorelementen entsteht beziehungsweise vorhanden ist. Dadurch kann eine Bestrahlung durch einheitliches Material und die Dosimetrie vereinfacht werden. Vorteilhaft kann deshalb auch vorgesehen sein, dass die äußere Oberfläche des Innenapplikatorelements im eingeführten Zustand, insbesondere im Bereich der abzugebenden Strahlendosen, zumindest bereichsweise an der Innenoberfläche des Aufnahmeraums des Außenapplikatorelements anliegt.

In weiterer Ausgestaltung kann im Aufnahmeraum des Außenapplikatorelements wenigstens ein Einlageelement aus Strahlen schirmendem Material zur wenigstens bereichsweisen Strahlabschirmung vorgesehen sein. Alternativ oder zusätzlich kann vorgesehen sein, dass das Außenapplikatorelement und/oder das Innenapplikatorelement zumindest bereichsweise aus einem Strahlen schirmendem Material zur wenigstens bereichsweisen Strahlabschirmung gebildet ist/sind. In diesem Fall ist das Strahlen schirmende Material direkt in das Außenapplikatorelement beziehungsweise das Innenapplikatorelement eingearbeitet. Auf diese Weise kann das äußere Applikatorelement beziehungsweise das innere Applikatorelement durch das Strahlen schirmende Material so abgeändert werden, dass er nicht mehr die gesamte Fläche, beispielsweise die gesamte Zylinderfläche, bestrahlt, sondern nur einen Teil davon. Damit kann Gewebe, welches nicht bestrahlt werden soll, von einer Bestrahlung ausgenommen werden. Beispielsweise kann auf diese Weise, je nach Wunsch und Anwendungsfall, eine Schirmung nach Vorn und/oder zur Seite erreicht werden. Handelt es sich bei dem Strahlen schirmenden Material um eine Einlage im Außenapplikatorelement, kann sich diese beispielsweise über die gesamte Länge des Außenapplikatorelements, oder aber nur teilweise in dem Außenapplikatorelement erstrecken. Beispielsweise kann eine solche Einlage als kreissegmentförmiges Bauteil, beispielsweise als halbkreisförmiges Rohr, ausgebildet sein. Handelt es sich bei dem Strahlen schirmenden Material um eine Einlage, kann diese vorteilhaft verschiebbar und/oder drehbar im Außenapplikatorelement angeordnet sein. Dabei ist die Erfindung weder auf eine bestimmte Anzahl von Einlageelementen, noch auf eine bestimmte Konfiguration der Einlageelemente, noch auf ein bestimmtes Strahlen schirmendes Material beschränkt.

Erfindungsgemäß sind das Innenapplikatorelement an seiner Spitze und/oder das Außenapplikatorelement an seiner Spitze geschlossen.

Die Strahlentherapievorrichtung wird zur Bestrahlung der Vagina oder des Rektums.

Die Strahlentherapievorrichtung weist zunächst eine Strahlungsquelleneinrichtung auf, mittels derer die zur Bestrahlung erforderlichen Strahlungsdosen erzeugt werden. Insbesondre ist die Strahlungsquelleneinrichtung zur Erezeugung von Strahlen für die Radiotherapie ausgebildet. An der Strahlungsquelleneinrichtung wird die Applikatoreinrichtung angeordnet.

Bevorzugt ist ferner eine Strahlentherapievorrichtung, bei der im Innenapplikatorelement eine Sondeeinrichtung oder ein Strahlungsquellenelement der Strahlungsquelleneinrichtung vorgesehen ist, wobei das Innenapplikatorelement mit der darin befindlichen Sondeneinrichtung oder dem darin befindlichen Strahlungsquellenelement verschiebbar und/oder drehbar im Außenapplikatorelement ausgebildet oder angeordnet ist.

Die Erfindung wird nun anhand von Ausführungsbeispielen unter Bezugnahme auf die beiliegenden Zeichnungen näher erläutert. Es zeigen
- Figur 1: eine perspektivische Darstellung einzelner Bauteile der erfindungsgemäßen Strahlentherapievorrichtung und der erfindungsgemäßen Applikatoreinrichtung;
- Figur 2: eine Darstellung der erfindungsgemäßen Strahlentherapievorrichtung gemäß Figur 1 in zusammengebautem Zustand; und
- Figur 3: eine Schnittdarstellung der Strahlentherapievorrichtung gemäß Figur 2.

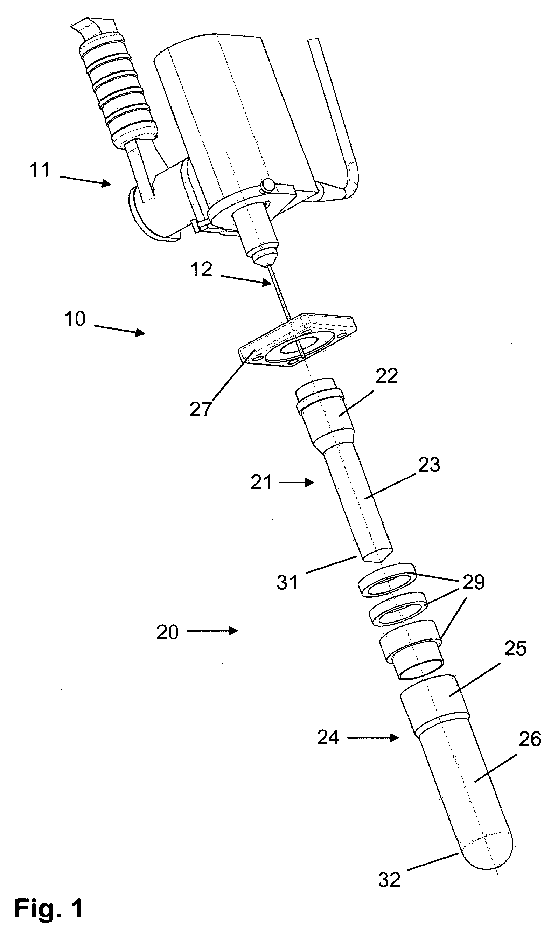

In den Figuren 1 bis 3 ist eine erfindungsgemäße Strahlentherapievorrichtung 10 dargestellt. Die Strahlentherapievorrichtung 10 weist eine
Strahlungsquelleneinrichtung 11 auf, die insbesondere zur Erzeugung von Strahlung für die Radiotherapie ausgebildet ist. Zum Applizieren der erzeugten Strahlung ist ein Strahlungsquellenelement 12 vorgesehen.

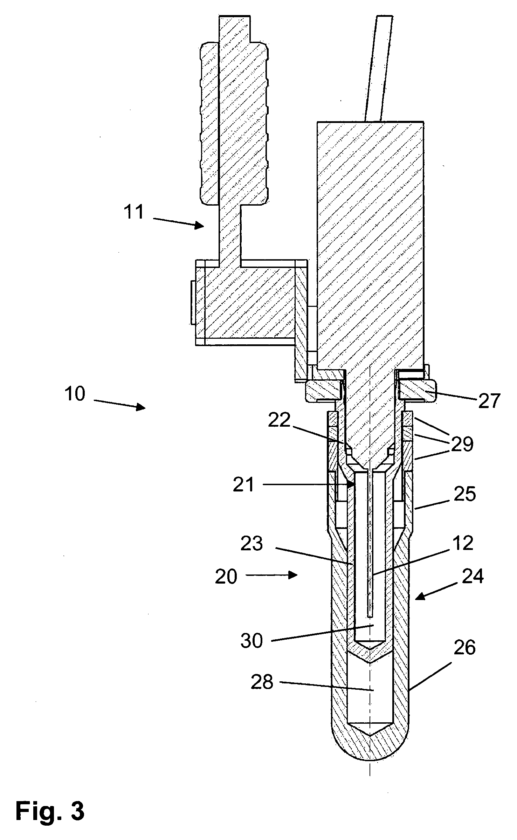

In den Figuren 1 bis 3 ist weiterhin eine erfindungsgemäße Applikatoreinrichtung 20 dargestellt. Diese wird zur Bestrahlung von Körperhöhlen eingesetzt, beispielsweise zur Bestrahlung der Vagina oder des Rektums. Die Applikatoreinrichtung 20 besteht zunächst aus einem inneren Applikatorelement 21. Dieses weist einen Fußbereich 22 zur Aufnahme von Elementen der Strahlentherapievorrichtung 10 auf. Weiterhin verfügt das Innenapplikatorelement 21 über einen sich an den Fußbereich 22 anschließenden zylindrischen Führungsbereich 23. Zusätzlich weist die Applikatoreinrichtung 20 ein äußeres Applikatorelement 24 auf. Auch das Außenapplikatorelement 24 weist einen Fußbereich 25 und einen sich daran anschließenden zylindrischen Führungsbereich 26 auf. Darüber hinaus verfügt die Applikatoreinrichtung 20 noch über eine Befestigungs- und/oder Fixiereinrichtung 27 zur Befestigung an einem Patienten. Diese Einrichtung 27 kann je nach Ausgestaltung Bestandteil des Außenapplikatorelements 24 oder des Innenapplikatorelements 21 sein. Zusätzlich sind Abstandhalterelemente 29 vorgesehen, die sich im zusammengebauten Zustand im Bereich des Fußbereichs 22 des Innenapplikatorelements 23 befinden, so wie dies insbesondere in Figur 3 dargestellt ist.

Das Innenapplikatorelement 21 und das Außenapplikatorelement 24 sind an den Spitzen 31, 32 geschlossen.

Das Innenapplikatorelement 21 ist über seinen Fußbereich 22 an der Strahlungsquelleneinrichtung 11 befestigt. Dadurch wird ermöglicht, dass das Strahlungsquellenelement 12 und das Innenapplikatorelement 21 gemeinsam im Außenapplikatorelement 24 bewegt werden können.

Dabei kann das Innenapplikatorelement 11 mit dem darin befindlichen Strahlungsquellenelement 12 verschiebbar und/oder drehbar im Außenapplikatorelement 24 ausgebildet oder angeordnet sein.

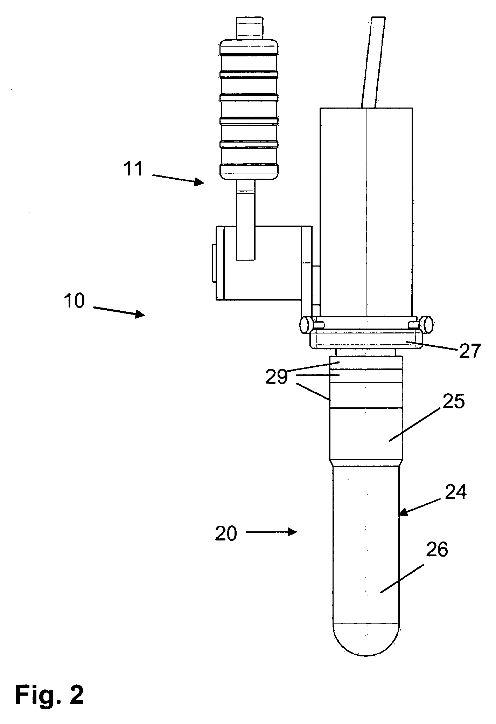

Die in Figur 1 dargestellte Applikatoreinrichtung 20 ist Bestandteil der Strahlentherapievorrichtung 10. die in den Figuren 2 und 3 in zusammengebautem Zustand dargestellt ist. In Figur 2 ist allgemein dargestellt, dass die Strahlentherapievorrichtung 10 aus der Strahlungsquelleneinrichtung 11 besteht, in der eine Strahlung, beispielsweise eine Strahlung für eine Radiotherapie, erzeugt wird. An der Strahlungsquelleneinrichtung 11 angeordnet ist die in Figur 1 dargestellte Applikatoreinrichtung 20. Wie dies vorteilhaft realisiert sein kann, ist im Zusammenhang mit Figur 3 dargestellt.

Die Applikatoreinrichtung 20 besteht aus dem inneren Applikatorelement 21 und dem äußeren Applikatorelement 24. Das äußere Applikatorelement 24 wird in eine zu bestrahlende Körperhöhle eingebracht und über die Befestigungseinrichtung 27, beispielsweise eine Riemenkonstruktion, am Patienten befestigt und in einer bestimmten Position fixiert. Dann kann beispielsweise mittels Ultraschall oder Röntgen kontrolliert werden, ob das Außenapplikatorelement 24 an der richtigen Stelle sitzt.

Das innere Applikatorelement 21 wird über ein Strahlungsquellenelement 12 der Strahlungsquelleneinrichtung 11 geschoben und anschließend in das äußere Applikatorelement 24 eingeschoben, Dabei weist das Innenapplikatorelement 21 einen Aufnahmeraum 30 für das Strahlungsquellenelement 12 auf, während im Außenapplikatorelement 24 ein entsprechender Aufnahmeraum 28 für das Innenapplikatorelement 21 vorgesehen ist. In Figur 3 ist dargestellt, dass sowohl der Fußbereich 22 des Innenapplikatorelements 21 in den Fußbereich 25 des Außenapplikatorelements 24, als auch der Führungsbereich 23 des Innenapplikatorelements 21 in den Führungsbereich 26 des Außenapplikatorelements 24 eingeschoben wird. Die Strahlendosen werden vorteilhaft jedoch nur im Bereich der Führungsbereiche 23 und 26 abgegeben. Das Innenapplikatorelement 21 und das Außenapplikatorelement 24 können über ihre jeweiligen Fußbereiche 22, 25 an der Strahlungsquelleneinrichtung 11 befestigt sein beziehungsweise werden.

Nach Einführung des Innenapplikatorelements 21 in das Außenapplikatorelement 24 kann die gewünschte Strahlendosis appliziert werden. Soll eine weitere Dosis an einer anderen Stelle appliziert werden, kann das innere Applikatorelement 21 in Längsrichtung verschoben werden, so dass sich insbesondere der Führungsbereich 23 des Innenapplikatorelements 21 innerhalb des Führungsbereichs 26 des Außenapplikatorelements 24 verschiebt.

Soll eine weitere Dosis an einer anderen Stelle appliziert werden, kann das innere Applikatorelement 21 in Längsrichtung verschoben werden. Dabei kann die zu verstellende Distanz mittels einzulegender Abstandhalterelemente 29 eingestellt werden. Die Abstandhalterelemente 29 sind vorteilhaft so geformt, dass ein Herausziehen des Innenapplikatorelements 21 unnötig ist. Dadurch wird vermieden, dass das äußere Applikatorelement 24 verrutscht. Um dies zu erreichen, können die Abstandhalterelemente 29 beispielsweise als Halbkreis oder 5/8-Kreis ausgebildet sein.

Durch wenigstens eine Einlage aus Strahlen schirmendem Material (nicht dargestellt) kann das Außenapplikatorelement 24 so abgeändert werden, dass es nicht mehr die gesamte Fläche, beispielsweise die gesamte Zylinderfläche, bestrahlt, sondern nur einen Teil davon. Damit kann Gewebe, welches nicht bestrahlt werden soll, von einer Bestrahlung ausgenommen werden. Alternativ oder zusätzlich kann auch vorgesehen sein, dass das Außenapplikatorelement 24 und/oder das Innenapplikatorelement 21 zumindest bereichsweise aus einem Strahlen schirmendem Material zur wenigstens bereichsweisen Strahlabschirmung gebildet ist/sind.

### Bezugszeichenliste

- 10: Strahlentherapievorrichtung
- 11: Strahlungsquelleneinrichtung
- 12: Strahlungsquellenelement

- 20: Applikatoreinrichtung
- 21: Innenapplikatorelement
- 22: Fußbereich
- 23: Führungsbereich
- 24: Außenapplikatorelement
- 25: Fußbereich
- 26: Führungsbereich
- 27: Befestigungs- und/oder Fixiereinrichtung
- 28: Aufnahmeraum des Außenapplikatorelements
- 29: Abstandhalterelement
- 30: Aufnahmeraum des Innenapplikatorelements
- 31: Spitze des Innenapplikatorelements
- 32: Spitze des Außenapplikatorelements

## Patentansprüche

1. Strahlentherapievorrichtung (10) für die Radiotherapie mit Röntgenstrahlung wobei die Strahlentherapievorrichtung (10) zur Bestrahlung der Vagina oder des Rektums eingesetzt wird, mit einer Strahlungsquelleneinrichtung (11) und mit einer Applikatoreinrichtung (20) zur Aufnahme einer Sondeneinrichtung oder eines Strahlungsquellenelements (12) der Strahlungsquelleneinrichtung (11), wobei die Applikatoreinrichtung (20) als Vaginalapplikator oder als Rektalapplikator ausgebildet ist, wobei die Applikatoreinrichtung (20) ein erstes Applikatorelement aufweist, welches als Innenapplikatorelement (21) ausgebildet ist, wobei die Sondeneinrichtung oder das Strahlungsquellenelement (12) der Strahlungsquelleneinrichtung (11) im Innenapplikatorelement (21) vorgesehen ist, wobei das Innenapplikatorelement (21) einen Fußbereich (22), über den das Innenapplikatorelement (21) an der Strahlungsquelleneinrichtung (11) befestigt ist, einen sich an den Fußbereich (22) anschließenden zylindrischen Führungsbereich (23) und eine dem Fußbereich (22) gegenüberliegende Spitze (31) aufweist, wobei die Applikatoreinrichtung (20) ein zweites Applikatorelement aufweist, das in Bezug auf das Innenapplikatorelement (21) als Außenapplikatorelement (24) ausgebildet ist, wobei das Außenapplikatorelement (24) einen Aufnahmeraum (28) zur Aufnahme des Innenapplikatorelements (21) aufweist, wobei das Außenapplikatorelement (24) dazu einen zylindrischen Führungsbereich (23, 26) aufweist und wobei der zylindrische Führungsbereich (23) des Innenapplikatorelements (21) in den zylindrischen Führungsbereich (26) des Außenapplikatorelements (24) eingeführt ist, und wobei das Innenapplikatorelement (21) an seiner Spitze (31) und das Außenapplikatorelement (24) an seiner Spitze (32) geschlossen sind.

2. Strahlentherapievorrichtung (10) nach Anspruch 1, **dadurch gekennzeichnet, dass** das Innenapplikatorelement (21) mit der darin befindlichen Sondeneinrichtung oder dem darin befindlichen Strahlungsquellenelement (12) verschiebbar und/oder drehbar im Außenapplikatorelement (24) ausgebildet oder angeordnet ist.

3. Strahlentherapievorrichtung (10) nach einem der Ansprüche 1 oder 2, **dadurch gekennzeichnet, dass** die Applikatoreinrichtung (20) eine Befestigungseinrichtung und/oder eine Fixiereinrichtung (27) aufweist.

4. Strahlentherapievorrichtung (10) nach einem der Ansprüche 1 bis 3, **dadurch gekennzeichnet, dass** in der Applikatoreinrichtung (20) wenigstens ein Abstandhalterelement (29) zur Fixierung des Innenapplikatorelements (21) in einer bestimmten Position vorgesehen ist.

5. Strahlentherapievorrichtung (10) nach einem der Ansprüche 1 bis 4, **dadurch gekennzeichnet, dass** die Applikatoreinrichtung (20) zwei oder mehr Innenapplikatorelemente (21) mit unterschiedlichen Längen, insbesondere mit unterschiedlichen Längen der zylindrischen Führungsbereiche (23), aufweist.

6. Strahlentherapievorrichtung (10) nach einem der Ansprüche 1 bis 5, **dadurch gekennzeichnet, dass** die äußere Oberfläche des Innenapplikatorelements (21) im eingeführten Zustand an der Innenoberfläche des Aufnahmeraums (28) des Außenapplikatorelements (24) anliegt.

7. Strahlentherapievorrichtung (10) nach einem der Ansprüche 1 bis 6, **dadurch gekennzeichnet, dass** im Aufnahmeraum (28) des Außenapplikatorelements (24) wenigstens ein Einlageelement aus Strahlen schirmendem Material zur wenigstens bereichsweisen Strahlabschirmung vorgesehen ist und/oder dass das Außenapplikatorelement (24) und/oder das Innenapplikatorelement (21) zumindest bereichsweise aus einem Strahlen schirmendem Material zur wenigstens bereichsweisen Strahlabschirmung gebildet ist/sind.

## Claims

1. Radiotherapy device (10) for radiation therapy with X-rays, wherein the radiotherapy device (10) is used for irradiating the vagina or the rectum, with a radiation source (11) and with an applicator (20) for receiving a probe or a radiation source element (12) of the radiation source (11), wherein the applicator (20) is designed as a vaginal applicator or as a rectal applicator, wherein the applicator (20) has a first applicator element designed as an inner applicator element (21), wherein the probe or the radiation source element (12) of the radiation source (11) is provided in the inner applicator element (21), wherein the inner applicator element (21) has a foot area (22), via which the inner applicator element (21) is secured on the radiation source (11), a cylindrical guide area (23) adjoining the foot area (22), and a tip (31) lying opposite the foot area (22), wherein the applicator (20) has a second applicator element, which is designed as an outer applicator element (24) in relation to the inner applicator element (21), wherein the outer applicator element (24) has a receiving space (28) for receiving the inner applicator element (21), wherein the outer applicator element (24) for this purpose has a cylindrical guide area (23, 26), and wherein the cylindrical guide area (23) of the inner applicator element (21) is inserted into the cylindrical guide area (26) of the outer applicator element (24), and wherein the inner applicator element (21) is closed at its tip (31) and the outer applicator element (24) is closed at its tip (32).

2. Radiotherapy device (10) according to Claim 1, **characterized in that** the inner applicator element (21), with the probe located therein or the radiation source element (12) located therein, is designed or arranged to be slidable and/or rotatable in the outer applicator element (24).

3. Radiotherapy device (10) according to either of Claims 1 and 2, **characterized in that** the applicator (20) has a fastening means and/or a fixing means (27).

4. Radiotherapy device (10) according to one of Claims 1 to 3, **characterized in that** at least one spacer element (29) is provided in the applicator (20) for the purpose of fixing the inner applicator element (21) in a defined position.

5. Radiotherapy device (10) according to one of Claims 1 to 4, **characterized in that** the applicator (20) has two or more inner applicator elements (21) with different lengths, in particular with different lengths of the cylindrical guide areas (23).

6. Radiotherapy device (10) according to one of Claims 1 to 5, **characterized in that** the outer surface of the inner applicator element (21), in the inserted state, bears on the inner surface of the receiving space (28) of the outer applicator element (24).

7. Radiotherapy device (10) according to one of Claims 1 to 6, **characterized in that** at least one insert element made of radiation-screening material is provided in the receiving space (28) of the outer applicator element (24), for the purpose of at least partial radiation screening, and/or **in that** the outer applicator element (24) and/or the inner applicator element (21) are/is at least partially made of a radiation-screening material, for the purpose of at least partial radiation screening.

## Revendications

1. Dispositif de radiothérapie (10) destiné à effectuer une radiothérapie par rayons X, dans lequel le dispositif de radiothérapie (10) est utilisé pour exposer le vagin ou le rectum au moyen d'un dispositif de source de rayonnement (11) et au moyen d'un dispositif applicateur (20) permettant de recevoir un dispositif de sondage ou un élément de source de rayonnement (12) du dispositif de source de rayonnement (11), dans lequel le dispositif applicateur (20) est réalisé sous la forme d'un applicateur vaginal ou d'un applicateur rectal, dans lequel le dispositif applicateur (20) comporte un premier élément applicateur qui est réalisé sous la forme d'un élément applicateur interne (21), dans lequel le dispositif de sondage ou l'élément de source de rayonnement (12) du dispositif de source de rayonnement (11) est disposé dans l'élément applicateur interne (21), dans lequel l'élément applicateur interne (21) comporte une région de pied (22) par l'intermédiaire de laquelle l'élément applicateur interne (21) est fixé au dispositif de source de rayonnement (11), une région de guidage (23) cylindrique se raccordant à la région de pied (22) et une pointe (31) opposée à la région de pied (22), dans lequel le dispositif applicateur (20) comporte un deuxième élément applicateur qui est réalisé sous la forme d'un élément applicateur externe (24) par rapport à l'élément applicateur interne (21), dans lequel l'élément applicateur externe (24) comporte un espace de réception (28) destiné à recevoir l'élément applicateur interne (21), dans lequel l'élément applicateur externe (24) comporte à cet effet une région de guidage cylindrique (23, 26) et dans lequel la région de guidage cylindrique (23) de l'élément applicateur interne (21) est introduite dans la région de guidage cylindrique (26) de l'élément applicateur externe (24), et dans lequel l'élément applicateur interne (21) est fermé au niveau de sa pointe (31) et l'élément applicateur externe (24) est fermé au niveau de sa pointe (32).

2. Dispositif de radiothérapie (10) selon la revendication 1, **caractérisé en ce que** l'élément applicateur interne (21) est réalisé ou peut être disposé de manière à pouvoir être décalé et/ou mis en rotation dans l'élément applicateur externe (24) avec le dispositif de sondage qui se trouve dans celui-ci ou avec l'élément de source de rayonnement (12) qui se trouve dans celui-ci.

3. Dispositif de radiothérapie (10) selon la revendication 1 ou 2, **caractérisé en ce que** le dispositif applicateur (20) comporte un dispositif de fixation et/ou un dispositif d'immobilisation (27).

4. Dispositif de radiothérapie (10) selon l'une quelconque des revendications 1 à 3, **caractérisé en ce qu'**il est prévu dans le dispositif applicateur (20) au moins un élément d'espacement (29) destiné à immobiliser l'élément applicateur interne (21) à une position déterminée.

5. Dispositif de radiothérapie (10) selon l'une quelconque des revendications 1 à 4, **caractérisé en ce que** le dispositif applicateur (20) comporte deux éléments applicateurs internes (21) ou plus, ayant des longueurs différentes et plus particulièrement, ayant des longueurs différentes de la région de guidage cylindrique (23).

6. Dispositif de radiothérapie (10) selon l'une quelconque des revendications 1 à 5, **caractérisé en ce que** la surface extérieure de l'élément applicateur interne (21) à l'état introduit appuie sur la surface interne de l'espace de réception (28) de l'élément applicateur externe (24).

7. Dispositif de radiothérapie (10) selon l'une quelconque des revendications 1 à 6, **caractérisé en ce qu'**au moins un élément d'insert constitué d'un matériau opaque au rayonnement est prévu dans l'espace de réception (28) de l'élément applicateur (24) pour occulter le rayonnement au moins par régions et/ou **en ce que** l'élément applicateur externe (24) et/ou l'élément applicateur interne (21) est/sont constitué(s) au moins par régions d'un matériau opaque au rayonnement pour occulter le rayonnement au moins par régions.
